# EUROPÄISCHE PATENTSCHRIFT

(11) **EP 0 868 912 B1**
(45) Veröffentlichungstag und Bekanntmachung des Hinweises auf die Patenterteilung: **11.12.2002**
(21) Anmeldenummer: 98103625.4
(22) Anmeldetag: 02.03.1998
(51) Int. Cl.: A61K 9/52, A61K 9/32, C05G 5/00, A01N 25/26

(54) **Verwendung von redispergierbaren Polymerpulvern oder Polymergranulaten zum Überziehen von pharmazeutischen oder agrochemischen Darreichungsformen**
Use of redispersible polymer powders or polymer granulates for coating pharmaceutical or agrochemical delivery forms
Utilisation de poudres polymères ou de granulés polymères pour enrober des formes d'administration pharmaceutiques ou agrochimiques

(30) Priorität: 10.03.1997 DE 19709532
(43) Veröffentlichungstag der Anmeldung: 07.10.1998
(73) Patentinhaber: BASF AKTIENGESELLSCHAFT, 67056 Ludwigshafen (DE)
(72) Erfinder: Kolter, Karl, Dr., 67117 Limburgerhof (DE); Tiefensee, Kristin, Dr., 67368 Westheim (DE); Stadler, Reinhold, Dr., 67489 Kirrweilen (DE); Zeitz, Katrin, Dr., 67067 Ludwigshafen (DE)

(56) Entgegenhaltungen:
- BE-A- 662 884
- DE-B- 2 612 889
- US-A- 3 935 326

## Beschreibung

Die vorliegende Erfindung betrifft die Verwendung von redispergierbaren Polymerpulvern oder Polymergranulaten bestehend aus Polyvinylacetat und N-Vinylpyrrolidon-haltigen Polymeren zum Überziehen von pharmazeutischen oder agrochemischen Darreichungsformen, wobei die Freisetzung des Wirkstoffes aus der Darreichungsform exakt gesteuert werden kann.

Feste pharmazeutische Darreichungsformen wie Tabletten, Kapseln, Pellets, Granulate, Kristalle etc. werden aus sehr unterschiedlichen Gründen gecoatet, d. h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüberhinaus läßt sich insbesondere die Freisetzungsgeschwindigkeit des Wirkstoffes durch den Filmüberzug einstellen. In ähnlicher Weise gelten diese Kriterien auch für agrochemische Darreichungsformen.

Generell unterscheidet man Instant-Release-Formen und Retard-bzw. Slow-Release-Formen.

Bei Instant-Release-Formen soll der Wirkstoff in möglichst kurzer Zeit freigesetzt werden. Hierbei darf der Überzug die Freisetzung des Wirkstoffes aus dem Kern nicht oder nur wenig behindern. In der pharmazeutischen Anwendungstechnik sind Instant-Release-Formen Zubereitungen, bei denen mehr als 80 % des Wirkstoffs innerhalb einer Stunde freigesetzt werden.

Bei Retard-Formen ist hingegen die Freisetzung verzögert, um beispielsweise Plasmaspiegelspitzen und damit mögliche Nebenwirkungen zu verhindern oder die Einnahmefrequenz zu verrringern. Bei den sogenannten überzogenen Retard-Formen, auch Coating-Retard-Formen genannt, verlangsamt ein Filmüberzug die Freisetzung des Arzneistoffes. Hierzu werden häufig wasserunlösliche Cellulosederivate wie Ethylcellulose oder (Meth)acrylat Copolymere, insbesonders Eudragit® NE, RS oder RL (Röhm Pharma, Weiterstadt), eingesetzt. Für Eudragit® RS und RL werden Weichmacherzusätze von 10 bis 20 Gew.-%, bezogen auf den Filmbildner, empfohlen. Bei Ethylcellulose ist ein noch höherer Weichmacheranteil (ca. 30 Gew.-%) unabdingbar. Lediglich Eudragit® NE benötigt keinen Weichmacher, da es eine sehr niedrige Glasübergangstemperatur und Mindestfilmbildungstemperatur besitzt. Dadurch bedingt ist es allerdings klebrig und schwer zu verarbeiten.

In dem US-Patent 5,252,704 wird die Herstellung von redispergierbaren Polymerpulvern unter Verwendung von Polyvinylpyrrolidon als Dispergierhilfsstoff beschrieben. Als Einsatzgebiet ist die Anwendung in der Zementherstellung angegeben. Pharmazeutische Anwendungen hingegen sind nicht erwähnt.

Die DE-B-4341156 beschreibt die Verwendung von in Wasser dispergierbaren Kunststoffdispersionspulvern mit einer Kern-Hülle-Struktur als Arzneimittelträger, wobei bei der pharmazeutischen Verarbeitung lediglich ein trockenes Untermischen des Polymerpulvers unter den Arzneistoff und anschließendes Verpressen erfolgt. Solche sogenannten Matrixformen unterscheiden sich grundlegend von Coating-Retard-Formen.

In der DE-A-4220782 werden Verfahren zur Herstellung von festen pharmazeutischen Retardformen durch Auftragen eines Bindemittels auf einen wirkstoffhaltigen Kern beschrieben. Bei den hier verwendeten Bindemitteln handelt es sich um Polymere auf (Meth)acrylatbasis.

US-Patent 3,935,326 beschreibt die Herstellung fester überzogener Arzneiformen durch Auftragen einer wässerigen Dispersion, die einen hohen Anteil an Polyvinylacetat, jedoch einen sehr geringen Anteil an Polyvinylpyrrolidon aufweist.

Der vorliegenden Erfindung lag die Aufgabe zugrunde, Polymerpulver oder Polymergranulate, bestehend aus einem wasserunlöslichen und wasserlöslichen Polymer zu finden, die vollständig redispergierbar und zum gleichmäßigen Überziehen von pharmazeutischen und agrochemischen Darreichungsformen geeignet sind. Die auf diese Weise hergestellten Formen sollten eine leicht steuerbare Freisetzung der Wirkstoffe ermöglichen.

Die Aufgabe wurde erfindungsgemäß gelöst durch Verwendung von redispergierbaren Polymerpulvern oder Polymergranulaten aus
a) 10 bis 95 Gew.-% Polyvinylacetat,
b) 5 bis 90 Gew.-% eines N-Vinylpyrrolidon-haltigen Polymers,
c) 0 bis 20 Gew.-% eines weiteren wasserlöslichen oder wasserquellbaren Stoffes und
d) 0 bis 20 Gew.-% eines wasserunlöslichen Puderungsmittels sowie gegebenenfalls
e) weiteren Zusatzstoffen,
zum Überziehen von pharmazeutischen oder agrochemischen Darreichungsformen.

Mit Hilfe dieser Polymeren kann die Freisetzung des Wirkstoffes aus der Darreichungsform exakt gesteuert werden, so daß sowohl Instant-Release als auch Retard- oder Slow-Release-Formen zur Verfügung gestellt werden können.

Die Herstellung der redispergierbaren Polymerpulver erfolgt in der Weise, daß zunächst Vinylacetat emulsionspolymerisiert wird und zu der entstandenen scherstabilen und feinteiligen Dispersion das N-Vinylpyrrolidon-haltige Polymer sowie gegebenenfalls weitere Hilfsstoffe zugegeben werden und die Mischung sprühgetrocknet wird. Bevorzugte N-Vinylpyrrolidon-haltige Polymere sind beispielsweise Polyvinylpyrrolidon und Vinylacetat-Vinylpyrrolidon Copolymere.

Die K-Werte der Polymerisate sollen im Bereich von 10 bis 350, vorzugsweise 30 bis 150, besonders bevorzugt im Bereich von 50 und 90 liegen. Der jeweils gewünschte K-Wert läßt sich in an sich bekannter Weise durch die Wahl der Polymerisationsbedingungen, beispielsweise der Polymerisationsdauer und der Initiatorkonzentration einstellen. Die K-Werte werden nach Fikentscher, Cellulosechemie, Bd. 13, S. 58-64 und 71-74 (1932) bei 25°C in 0,1 Gew.-%iger wäßriger Lösung gemessen.

Der Zusatz von feinteiligen, wasserunlöslichen Sprühhilfsmitteln bei der Sprühtrocknung kann ein Verkleben der gebildeten Teilchen verhindern. Hierbei ist ein Einstäuben dieser Sprühhilfsmittel in den Sprühtrockner besonders vorteilhaft. Es ist aber auch möglich, die Sprühhilfsmittel bereits der zu versprühenden Dispersion zuzusetzen oder sie erst nach der Sprühtrocknung zur Verhinderung eines Zusammenbackens der Teilchen unterzumischen. Als Puderungsmittel können bis zu 20 Gew.-%, bezogen auf den Feststoffgehalt, feinteilige wasserunlösliche Stoffe, insbesondere mindestens ein Vertreter aus der Gruppe Cellulose, bevorzugt mikrokristalline Cellulose, disperse Kieselsäure, Talkum, Bentonit, Magnesiumstearat oder ein Calciumphosphat verwendet werden.

Die Emulsionspolymerisation wird in bekannter Weise bei Temperaturen von 50°C bis 95°C, bevorzugt von 60°C bis 80°C, in Gegenwart von bekannten Polymerisationsinitiatoren durchgeführt.

Als Polymerisationsinitiatoren kommen vorzugsweise Radikalbildner, beispielsweise Peroxide wie bevorzugt Peroxosulfate, Peroxodisulfate und Azoverbindungen wie Azodiisobutyronitril in Betracht.

Als Emulgatoren können sowohl ionische wie nicht-ionische Emulgatoren oder deren Mischungen eingesetzt werden. Ihre Gesamtkonzentration liegt zwischen 0,2 und 10 Gew.-%, bevorzugt zwischen 0,4 und 7 Gew.-%, bezogen auf den Gesamtmonomerengehalt. Ferner können als weitere Hilfsstoffe bis zu 20 Gew.-%, bezogen auf den Feststoffgehalt, wasserlösliche oder wasserquellbare Schutzkolloide eingesetzt werden, wie z.B. Cellulosederivate, bevorzugt Hydroxypropylmethylcellulose, Methylcellulose oder Hydroxyethylcellulose, Galactomannan, Pectin, Xanthan, Polyvinylalkohol, Acrylat-Methacrylat Copolymere, Natriumcarboxymethylstärke, Cellulose, abgebaute Stärken, Maltodextrine etc. Die emulgierenden Hilfsstoffe können dabei vor, während und nach der Polymerisation zugegeben werden.

Die Dispersion weist einen Feststoffgehalt von 10 bis 45 Gew.-%, bevorzugt von 15 bis 35 Gew.-% auf.

Die sedimentationsstabile Polymerdispersion weist mittlere Teilchengrößen von 0,1 bis 7 µm, bevorzugt von 0,3 bis 4 µm auf. Die Bestimmung erfolgt in der üblichen Weise z.B. mittels Ultrazentrifuge, Photonenkorrelationsspektroskopie oder durch Bestimmung der Lichtdurchlässigkeit. Die Partikelgröße wird üblicherweise über die Emulgatorkonzentration bzw. die Temperaturführung gesteuert.

Eine entscheidende Bedeutung kommt der Scherstabilität der Polyvinylacetat-Dispersion zu. Nur sehr scherstabile Dispersionen sind in der Lage, nach dem Versprühen ausreichend redispergierbare Pulver zu bilden.

Die Prüfung der Scherstabilität erfolgt durch Scherung der Dispersion mittels eines Noppenrührers bei 2000 U/min über 15 min und gravimetrische Bestimmung des Koagulates nach Siebung über ein 125 µm Sieb. Scherstabile Dispersionen weisen Koagulatanteile < 0,1% auf.

Die Zugabe des Polyvinylpyrrolidons erfolgt vorzugsweise als 10 bis 50 Gew.-%ige Lösung unter ständigem Rühren. Bei der Zugabe als Feststoff oder als höherkonzentrierte Lösung können Koagulationen auftreten.

Die Sprühtrocknung erfolgt in üblicher Weise in Sprühtürmen, wobei die zu trocknende Dispersion mittels Düsen oder Scheiben zerstäubt wird. Die Zuführung des heißen Gases, z. B. Luft oder Stickstoff, kann im Gleichstrom oder im Gegenstrom erfolgen, wobei das Gleichstromverfahren besonders bevorzugt ist, da es zu einer geringeren Temperaturbelastung führt.

Auch die FSD-Technologie (Fluidized Spray Drying), bei der zunächst sprühgetrocknet und sofort anschließend in der Wirbelschicht agglomeriert wird, kann eingesetzt werden. Alternativ kann auch gefriergetrocknet werden.

Das Gewichtsverhältnis von Polyvinylacetat zu Polyvinylpyrrolidon und/oder mindestens einem Copolymer aus Vinylacetat und Vinylpyrrolidon kann im Bereich von 95 : 5 bis 10 : 90 variiert werden, wodurch sich die Freisetzungseigenschaften verändern lassen. Bei steigendem Anteil von hydrophilem Polyvinylpyrrolidon beschleunigt sich die Freisetzung. Auf diese Weise kann die Freisetzung pharmazeutischer Wirkstoffe von sehr langsam (24 h) bis sehr schnell (0,25 h) eingestellt werden. Somit sind auch Instant-Release-Überzüge möglich, wofür sich bevorzugt Kombinationen mit einem Gewichtsverhältnis Polyvinylacetat zu Polyvinylpyrrolidon und/oder mindestens einem Copolymer aus Vinylacetat und Vinylpyrrolidon von < 70 : 30 eignen. Die Freisetzung kann darüber hinaus durch den Zusatz weiterer hydrophiler oder lipophiler Hilfsstoffe eingestellt werden. Der Zusatz hydrophiler Hilfsstoffe beispielsweise Hydroxypropylmethylcellulose, Hydroxypropylcellulose, Methylcellulose, Hydroxyethylcellulose, Galactomannan, Pectin, Xanthan, Polyvinylalkohol, Acrylat-Methacrylat Copolymere, Natriumcarboxymethylstärke, bzw. Cellulose, abgebaute Stärken, Maltodextrine etc. oder auch niedermolekularer Stoffe, wie z. B. Monosaccharide, Disaccharide, Zuckeralkohole, anorganische wasserlösliche Salze, Aminosäuren, wasserlösliche Säuren, bzw. deren Salze oder Tenside, beschleunigt die Freisetzung und kann die Dispersion zusätzlich stabilisieren.

Zusätzliche hydrophile Eigenschaften der erfindungsgemäßen, redispergierbaren Polymerpulver können durch Verwendung von teilweise hydrolysiertem Polyvinylacetat eingestellt werden, wobei hier insbesondere Polyvinylacetate mit einem Hydrolysegrad von bis zu 50 Mol-% verwendet werden.

Für Instant-Release-Zwecke kann somit insbesondere auch eine Kombination von Polyvinylacetat mit Polyvinylpyrrolidon und/oder mindestens einem Copolymer aus Vinylacetat und Vinylpyrrolidon in einem Gewichtsverhältnis von > 70 : 30 mit beispielsweise Hydroypropylmethylcellulose versetzt werden. Dabei ist es möglich die Hydroxypropylmethylcellulose während der Herstellung des redispergierbaren Polymers oder aber erst bei der Herstellung der Überzugsdispersion zuzusetzen. Besonders bevorzugt ist die Verwendung von Polyvinylalkohol und/oder Hydroxypropylmethylcellulose.

Das Aufbringen eines Retard-Überzugs kann unter Verwendung einer Lösung des Polymers in einem organischen Lösungsmittel oder bevorzugt einer 2 bis 50 Gew.-%igen, insbesondere einer 10 bis 30 Gew.-%igen Dispersion des Polymers in Wasser erfolgen. Aus anwendungstechnischen Gründen weist die wäßrige Verarbeitungsweise große Vorteile auf. Allerdings sind feinteilige Dispersionen thermodynamisch instabile Zubereitungen, die sehr empfindlich auf Streßfaktoren wie Temperaturschwankungen, Einfrieren, Scherung, Wasserverdunstung, Schütteln etc. reagieren und dadurch unbrauchbar werden. Überraschenderweise lassen sich diese geschilderten Nachteile durch Verwendung eines redispergierbaren Produktes bestehend aus Polyvinylacetat und Polyvinylpyrrolidon bzw. Vinylacetat-Vinylpyrrolidon Copolymeren sowie weiteren Formulierungshilfsmitteln eliminieren.

Die Herstellung der Dispersion zum Überziehen von pharmazeutischen oder agrochemischen Darreichungsformen erfolgt bevorzugt in der Weise, daß das redispergierbare Pulver bestehend aus Polyvinylacetat und Polyvinylpyrrolidon und/oder mindestens einem Copolymer aus Vinylacetat und Vinylpyrrolidon sowie eventuell hydrophilen oder lipophilen Zusatzstoffen in Wasser unter Rühren eingetragen wird. Anschließend können weitere für pharmazeutische Überzugszwecke übliche Hilfsstoffe wie wasserlösliche oder wasserunlösliche Farbstoffe (Farbpigmente), Weißpigmente (z. B. TᵢO₂), Antiklebemittel (z. B. Talkum, Glycerinmonostearat, Magnesiumstearat), Suspensionsstabilisatoren, Emulgatoren, Verdickungsmittel, Weichmacher etc. zugemischt werden. Es ist von Vorteil, diese Feststoffe vor der Einarbeitung in die Dispersion in Wasser aufzuschlämmen und mittels eines hochtourigen Rührers (Ultra-turrax) oder einer Mühle (Korundscheibenmühle) zu homogenisieren. Diese Coatingsuspension wird dann mittels geeigneter Geräte, wie z. B. Horizontaltrommelcoater, Dragierkessel, Wirbelschichtgeräte auf pharmazeutische Darreichungsformen aufgesprüht, wobei sich ein gleichmäßiger homogener Film ausbildet, der nach der Trocknung keiner weiteren Temperung oder Curing mehr bedarf. Filme mit einem hohen Anteil an Polyvinylacetat bleiben bei der Freisetzungsprüfung intakt und verlangsamen dadurch den Transport des Wirkstoffes in das Freisetzungsmedium; Filme mit niedrigem Anteil an Polyvinylacetat zerfallen bzw. dispergieren und der Arzneistoff wird schnell freigesetzt.

Bei den überzogenen Darreichungsformen handelt es sich bevorzugt um feste Zubereitungen. Darunter versteht man u.a. Tabletten, Mikrotabletten, Dragees, Pastillen, Kapseln, Kristalle, Granulate oder Pellets.

Anders als im pharmazeutischen Bereich, liegen die geforderten Freisetzungszeiten für Agrochemikalien z.B. im Pflanzenschutzbereich deutlich höher. Je nach Saison, Pflanzensorte und Bodenbeschaffenheit sind Freisetzungszeiten von 6 Wochen bis 6 Monaten gewünscht. Durch Variation der Schichtdicke, mit der das erfindungsgemäße Überzugsmittel auf den Wirkstoff aufgebracht wird sowie durch zusätzliche chemische Quervernetzung lassen sich die oben genannten, hohen Retardzeiten einstellen.

Als Pflanzenschutz-Wirkstoffe kommen herbizide, wachstumsregulatorische, insektizide und insbesondere fungizide Wirkstoffe in Betracht.

Bevorzugte feste Darreichungsformen im Pflanzenschutzbereich sind dabei Granulate und Pellets.

Die Bekämpfung von Schadpilzen mit den fungiziden Mitteln erfolgt zweckmäßig in der Weise, daß man eine fungizid wirksame Menge des fungiziden Mittels in oder auf dem Ackerboden, auf das im Ackerboden ausgebrachte Saatgut oder auf die sich daraus entwickelnden Pflanzen bzw. auf Sämlinge einwirken läßt.

Durch die verzögerte Freisetzung der Wirkstoffe kann die Freisetzungsrate der Wirkstoffe im Boden so gesteuert werden, daß etwa im Falle der fungiziden Pflanzenschutz-Wirkstoffe ein wirksamer Schutz vor Pilzkrankheiten über die gesamte Vegetationsperiode hinweg aufrechterhalten werden kann. Die Wirkstoffaufnahme erfolgt kontinuierlich über die Wurzeln in dem Maße der kontrollierten Freisetzung der Wirkstoffe aus den erfindungsgemäß formulierten Wirkstoffen, und die Wirkstoffe werden dann über die Wurzeln systemisch in den Pflanzen verteilt.

Gegenüber der zur Pilzbekämpfung verbreiteten Spritzapplikation der Pflanzenschutz-Wirkstoffe bietet das erfindungsgemäße Verfahren die folgenden Vorteile:
- Mit einer einzigen Ausbringung der erfindungsgemäß formulierten Wirkstoffe im Ackerboden, die vorteilhaft zusammen mit dem Saatgut bzw. mit der Einpflanzung von Sämlingen erfolgt, kann ein wirksamer Schutz der Pflanze etwa gegen Pilzkrankheiten über die gesamte Vegetationsperiode erzielt werden.
- Hierdurch entfällt die bisher übliche Anwendung von mehreren Spritzapplikationen in der wachsenden Kultur, wodurch ein erheblicher Arbeitsaufwand eingespart werden kann.
- Durch die Applikation der Pflanzenschutz-Wirkstoffe in Form der erfindungsgemäßen Formulierung ergeben sich geringere Mengen an den auszubringenden Wirkstoffen.
- Bei Anwendung des erfindungsgemäßen Verfahrens kann eine Saatgutbeizung entfallen.

Es ist ein wesentliches Merkmal der vorliegenden Erfindung, daß die Pflanzenschutz-Wirkstoffe in einer Formulierung mit verzögerter Wirkstoff-Freisetzung vorliegen. Durch eine solche kontrollierte Wirkstoff-Freisetzung ist es möglich, mit einer Ausbringung der erfindungsgemäßen fungiziden Mittel im Boden einen wirksamen Schutz der Kulturpflanzen gegen Pilzbefall über die gesamte Vegetationsperiode hinweg aufrechtzuerhalten.

Die Granulate werden als Umhüllungsgranulate hergestellt, indem die Wirkstoffe zunächst auf feste granulatförmige Trägerstoffe aufgetragen werden. Die erhaltenen wirkstoffhaltigen Granulate werden anschließend mit geeigneten Hüllsubstanzen umhüllt, die eine verzögerte kontrollierte Wirkstoff-Freigabe bewirken.

Geeignete feste Trägerstoffe für die Umhüllungsgranulate sind beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe, z.B. Crotonylidendiharnstoff, Isobutylidendiharnstoff und pflanzliche Produkte wie Getreidemehl, Maismehl, Baumrinden-, Holz- und Nußschalenmehl, Maisschrot, Cellulosepulver. Vorteilhaft werden Düngemittel als Trägerstoffe verwendet. Bevorzugt sind Düngemittelgranulate oder -pellets als Trägerstoff, und zwar insbesondere solche, die Phosphat enthalten.

Die Granulate weisen im allgemeinen einen Kornbereich von 0,1 bis 10 mm, vorzugsweise 0,5 bis 8 mm, insbesondere 1 bis 6 mm auf.

Das Aufbringen der Wirkstoffe auf die Trägerstoffe erfolgt in der Regel so, daß man sie in Form von Öl-in-Wasser-Emulsionen, Emulsionskonzentraten, Suspoemulsionen, Suspensionskonzentraten oder in organischen Lösungsmitteln oder vorzugsweise in Wasser gelöst aufsprüht.

Das Aufsprühen erfolgt z.B. in Wirbelbettapparaturen oder in Trommeln oder Drehtellern, in denen das Trägergranulat gerollt wird, in perforierten Kesseln mit kontrollierter Führung des Trocknungsmediums, zweckmäßig Luft, oder im Luftsuspensionsverfahren. Im allgemeinen werden für das Aufsprühen und das Trocknen Temperaturen zwischen 10°C und 110°C angewendet.

Der den aufgetragenen Wirkstoff enthaltende Trägerstoff wird anschließend mit den erfindungsgemäßen Polymermischungen umhüllt.

Die so erhaltenen und mit der Hüllschicht versehenen fungiziden Mittel können als solche für die erfindungsgemäße Bekämpfung von Pilzen unter kontrollierter Wirkstoff-Freigabe verwendet werden.

Es kann jedoch auch vorteilhaft sein, auf diese Mittel von außen zusätzlich Wirkstoff aufzutragen. Die so erhaltenen Mittel ermöglichen eine weitere Abstufung der kontollierten Wirkstoff-Freigabe, wobei die außen auf der Hüllschicht aufgetragenen Wirkstoffe für eine gezieltere Anfangswirkung von Bedeutung sind. Es kann zusätzlich von Vorteil sein, noch eine zweite Hüllschicht zu verwenden, wodurch sich für die verzögerte Freisetzung der Wirkstoffe eine weitere Steuerungsmöglichkeit ergibt.

Neben der vorstehend beschriebenen Umhüllungstechnik besteht eine weitere vorteilhafte Technik zur Herstellung der erfindungsgemäßen Mittel in einer Formulierung mit verzögerter Wirkstoff-Freisetzung darin, daß die Wirkstoffe in geeignete Matrixsubstanzen eingelagert werden, aus denen die Wirkstoffe verzögert und kontrolliert freigesetzt werden. Als Matrix kann hierbei beispielsweise das für die Umhüllung eingesetzte Material verwendet werden. Die Herstellung erfolgt dabei zweckmäßigerweise derart, daß die Wirkstoffe in der Lösung oder Dispersion des Hüllmaterials gelöst oder dispergiert werden und anschließend diese Zubereitung, wie vorstehend für die Hüllstoffe beschrieben, auf das Trägermaterial aufgebracht wird. Hierdurch wird sichergestellt, daß die Wirkstoffe gleichmäßig in der Hüllschicht verteilt werden. In der Regel ist die Freisetzung aus diesen Formulierungen diffusionskontrolliert.

Die fungiziden Mittel beispielsweise enthalten im allgemeinen zwischen 0,01 und 95, vorzugsweise 0,05 bis 90 Gew.-% Pflanzenschutz-Wirkstoff.

Die Aufwandmengen liegen je nach Art des gewünschten Effektes zwischen 0,02 und 5 kg, vorzugsweise 0,05 und 3 kg Pflanzenschutz-Wirkstoff pro ha. Dabei beträgt das Verhältnis der Wirkstoffe in einer fungiziden Zweier-Mischung im allgemeinen 50:1 bis 1:10, vorzugsweise 20:1 bis 1:5, insbesondere 10:1 bis 1:2.

Die fungiziden Mittel zeichnen sich durch eine hervorragende Wirksamkeit gegen ein breites Spektrum von pflanzenpathogenen Pilzen, insbesondere aus der Klasse der Ascomyceten und Basidiomyceten, aus.

Besondere Bedeutung haben sie für die Bekämpfung einer Vielzahl von Pilzen an verschiedenen Kulturpflanzen wie Getreide, z.B. Weizen, Roggen, Gerste, Hafer, Reis, Raps, Zuckerrüben, Mais, Soja, Kaffee, Zuckerrohr, Zierpflanzen und Gemüsepflanzen wie Gurken, Bohnen und Kürbisgewächsen. Mit besonderem Vorteil werden die erfindungsgemäß hergestellten Fungizide zur Pilzbekämpfung an Getreide verwendet.

Die Anwendung der Mittel erfolgt zweckmäßig in der Weise, daß man das Mittel im oder auf dem Boden auf das im Boden ausgebrachte Saatgut oder auf sich daraus entwickelnden Pflanzen bzw. auf Sämlinge einwirken läßt. Das Ausbringen des Mittels und das Ausbringen des Saatguts bzw. das Einpflanzen der Sämlinge kann in getrennten Arbeitsgängen erfolgen, wobei das Ausbringen des Mittels vor oder nach dem Ausbringen des Saatguts bzw. dem Einpflanzen der Sämlinge erfolgen kann.

Es ist besonders vorteilhaft, die formulierten Pflanzenschutz-Wirkstoffe zusammen mit dem Saatgut bzw. dem Einpflanzen der Sämlinge auszubringen.

In den nachfolgenden Beispielen wird die Herstellung und Verwendung der erfindungsgemäßen Polymere näher erläutert.

### Beispiel 1:

Herstellung eines redispergierbaren Pulvers aus Polyvinylacetat : Polyvinylpyrrolidon im Gewichtsverhältnis 80:20 und 5 Gew.-% Polyvinylalkohol.

In einem Rührgefäß mit Rückflußkühler wurden 500 g Vinylacetat mit 0,5 Gew.-% Natriumlaurylsulfat unter Stickstoff in Wasser emulgiert und durch Initiierung mit 0,3 Gew.-% Natriumpersulfat bei 75°C polymerisiert. Nach Zugabe von 5 Gew.-% Polyvinylalkohol (Mowiol® 8-88 der Fa. Hoechst) wurde das Reaktionsgemisch auf Raumtemperatur abgekühlt. Man erhielt eine Dispersion mit einem Feststoffgehalt von 27 Gew.-% und einer mittleren Teilchengröße von 1 µm.

Die Dispersion wurde mit 417 g einer 30 Gew.-%igen wässrigen Polyvinylpyrrolidon-Lösung (Kollidon® K 30, der Fa. BASF) versetzt und die Mischung im Stickstoffgleichstrom bei einer Eingangstemperatur von 140°C sprühgetrocknet. Man erhielt ein weißes, frei fließendes Pulver.

### Beispiel 2

### Retard-Überzug auf Theophyllinpellets

Die Retard-Überzugsdispersion wies folgende Zusammensetzung auf:

| | |
|---|---|
| Titandioxid (Fa. Kronos) | 0,5 Gew.-% |
| Talkum (Fa. Riedel-de-Haen) | 4,0 Gew.-% |
| Sicovit® rot 30 (Fa. BASF) | 0,5 Gew.-% |
| redispergierbares Pulver, bestehend aus Polyvinylacetat und Polyvinylpyrrolidon im Gewichtsverhältnis 80:20 mit 5,0 Gew.-% Polyvinylalkohol (Mowiol® 8-88, Fa. Hoechst) | 15,0 Gew.-% |
| Wasser | 80.0 Gew.-% |

Der Gehalt an Feststoff betrug 20 Gew.-%.

Zur Herstellung wurde zunächst das redispergierbare Pulver in 70 % der Wassermenge unter Rühren eingetragen und 15 Minuten gerührt. Separat wurde aus Titandioxid, Talkum und Sicovit® rot 30 und 10 % der Wassermenge eine Pigmentsuspension hergestellt, die nach Passage einer Korundscheibenmühle mit der redispergierten Polymerdispersion vereinigt wurde. 671,0 g (inkl. 10 % Zuschlag für Sprühverluste) dieser Coatingdispersion wurden in einem Aeromatic Strea 1 (Fa. Aeromatic) in der Wirbelschicht auf 500 g Theophyllinpellets (Spherofillin 0,8 bis 1,3 mm, Fa. Knoll AG) aufgesprüht.

Der Coatingprozeß wurde unter folgenden Bedingungen durchgeführt:

| | |
|---|---|
| Düse | 0,8 mm |
| Vorwärmen | 45°C/5 min |
| Zuluftmenge | 100-130 m³/h |
| Zulufttemperatur | 45°C |
| Ablufttemperatur | ca. 30°C |
| Sprühart | kontinuierlich |
| Sprühdruck | 0,8 bar |
| Sprührate | 8,5 ml/min |
| Sprühzeit | ca. 80 min |
| Trocknen | 60°C/5 min |

Es bildete sich ein gleichmäßiger, glatter Überzug aus.

Zur Bestimmung der Freisetzung wurden die überzogenen Pellets, entsprechend einer Menge von 300 mg Theophyllin, in Kapseln gefüllt und diese in jeweils 900 ml künstlichem Magensaft eingebracht. Die Freisetzung erfolgte in einer Paddle-Apparatur (Fa. Pharmatest) bei 37°C und 50 U/min. Nach 2 Stunden wurde durch Zugabe eines Phosphatpufferkonzentrates umgepuffert auf pH 6,8.

Folgende Freisetzungswerte wurden erreicht:

| | |
|---|---|
| 1 h | 14,5 % |
| 2 h | 27,5 % |
| 4 h | 37,4 % |
| 8 h | 63,4 % |
| 12 h | 79,4 % |
| 16 h | 94,0 % |
| 20 h | 100,0 % |

Die Freisetzung war nach 6 monatiger Lagerung bei 30°C/70% rel. Feuchte und 40°C/75% rel. Feuchte unverändert.

### Beispiel 3

### Retard-Überzug auf Coffeinpellets

Die Retard-Überzugsdispersion wies folgende Zusammensetzung auf:

| | |
|---|---|
| redispergierbares Pulver, bestehend aus Polyvinylacetat und Polyvinylpyrrolidon im Gewichtsverhältnis 80:20 | 20,0 Gew.-% |
| Talkum | 5,0 Gew.-% |
| Wasser | 75,0 Gew.-% |

Der Gehalt an Feststoff betrug 25 Gew.-%. Zur Herstellung wurde Talkum in der vorgegebenen Wassermenge mittels eines Ultra-turrax fein dispergiert. Anschließend wurde das redispergierbare Pulver unter Rühren mit einem Flügelrührer langsam eingetragen und noch 30 Minuten weitergerührt.

700,0 g dieser Überzugsdispersion wurden in einem Aeromatic Strea 1 (Fa. Aeromatic) in der Wirbelschicht auf 500 g Coffeingranulat (0,5-1,0 mm) aufgesprüht.

Der Coatingprozeß wurde unter folgenden Bedingungen durchgeführt.

| | |
|---|---|
| Düse | 0,8 mm |
| Vorwärmen | 45°C/5 min |
| Zuluftmenge | 100-130 m³/h |
| Zulufttemperatur | 50°C |
| Ablufttemperatur | ca. 30°C |
| Sprühart | kontinuierlich |
| Sprühdruck | 0,8 bar |
| Sprührate | 10 ml/min |
| Sprühzeit | ca. 70 min |
| Trocknen | 60°C/5 min |

Es bildete sich ein gleichmäßiger Überzug aus.

Zur Bestimmung der Freisetzung wurden überzogene Pellets entsprechend einer Menge von 100 mg Coffein in Kapseln gefüllt und diese in jeweils 900 ml künstlichem Magensaft eingebracht. Die Freisetzung erfolgte in einer Paddle-Apparatur (Fa. Pharmatest) bei 37°C und 50 U/min. Nach 2 Stunden wurde durch Zugabe eines Phosphatpufferkonzentrates umgepuffert auf pH 6,8.

Folgende Freisetzungswerte wurden erreicht:

| | |
|---|---|
| 1 h | 8,3 % |
| 2 h | 16,4 % |
| 4 h | 40,6 % |
| 8 h | 74,9 % |
| 16 h | 97,2 % |

Die Freisetzung war nach 6 monatiger Lagerung bei 30°C/70% rel. Feuchte und 40°C/75% rel. Feuchte unverändert.

### Beispiel 4

### Instant-Release-Überzug auf Propranolol-Tabletten

5 000 g Propranolol-HCl-Tabletten mit folgender Zusammensetzung

| | |
|---|---|
| Propranolol-HCl | 40,0 mg |
| Ludipress® (Fa. BASF) | 195,0 mg |
| Kollidon® VA 64 (Fa. BASF) | 12,5 mg |
| Magnesiumstearat | 2,5 mg |
| Gesamtgewicht | 250,0 mg |

mit einem Gewicht von 250 mg, 9 mm Durchmesser, gewölbte Form wurden in einer Accela-Cota 24" (Fa. Manesty) mit 1 260 g (inkl. 10 % Zuschlag für Sprühverluste) einer Coatingsuspension folgender Zusammensetzung besprüht:

| | |
|---|---|
| redispergierbares Pulver, bestehend aus Polyvinylacetat und Polyvinylpyrrolidon im Gewichtsverhältnis 50:50 mit 5 Gew.-% Polyvinylalkohol (Mowiol® 8/88, Fa. Hoechst) und 1 Gew.-% hochdisperse Kieselsäure | 10,0 Gew.-% |
| Lutrol® E 6000 (Fa. BASF) | 1,0 Gew.-% |
| Sicovit® rot 30 (Fa. BASF) | 1,5 Gew.-% |
| Titandioxid (Fa. Kronos) | 3,0 Gew.-% |
| Talkum (Fa. Riedel-de-Haen) | 4,5 Gew.-% |
| Wasser | 80,0 Gew.-% |

Die Herstellung der Coatingsuspension erfolgte in analoger Weise wie unter Beispiel 2 beschrieben.

Der Feststoffgehalt der Coatingsuspension betrug 20 Gew.-% und die Auftragsmenge lag bei 6 mg Feststoff/cm² Tablettenoberfläche. Das Coaten der Tablettenkerne erfolgte unter folgenden Gerätebedingungen:

| | |
|---|---|
| Kessel | 12 U/min |
| Düse | 1,0 mm |
| Vorwärmen | 60°C/5 min |
| Sprühdruck | 2,0 bar |
| Sprühart | kontinuierlich |
| Differenzdruck | - 2 mm |
| Ablufttemperatur | ca. 40°C |
| Zulufttemperatur | 60°C |
| Sprührate | 25 g/min |
| Trocknen | 60°C/5 min |

Die Filmtabletten wiesen eine glatte, gleichmäßige Oberfläche auf, wobei die Gravur in keinster Weise verschmiert wurde.

Der Zerfall der Filmtabletten in künstlichem Magensaft lag bei 7 Minuten und entsprach damit den Forderungen des Deutschen Arzneibuches für Instant-Release-Formen (< 15 Minuten).

Bei der Freisetzung in künstlichem Magensaft mittels der Paddle-Apparatur bei 37°C und 50 U/min wurden innerhalb 30 Minuten mehr als 90 der Arzneistoffmenge freigesetzt.

Freisetzung:

| | |
|---|---|
| 30 min | 96,5 % |
| 60 min | 99,5 % |

### Beispiel 5

### Instant-Release-Überzug auf Propranolol-Tabletten

Rezeptur und Verarbeitung erfolgten analog Beispiel 4, jedoch mit einem redispergierbaren Pulver aus Polyvinylacetat und Polyvinylpyrrolidon im Gewichtsverhältnis 70:30. Der Coatingsuspension wurde zusätzlich 3 Gew.-% Hydroxypropylmethylcellulose (Pharmacoat® 606, Fa. Shin-etsu) zugefügt.

Die Filmtabletten wiesen eine glatte, gleichmäßige Oberfläche auf mit schöner Ausformung der Gravur.
Der Zerfall lag bei 6 Minuten und die Freisetzung nach 30 Minuten bei 98,1 %.

### Beispiel 6

In einem Rührgefäß wurden unter Rühren 220 g redispergierbares Pulver, bestehend aus Polyvinylacetat und Polyvinylpyrrolidon (Kollidon® 30, Fa. BASF) im Gewichtsverhältnis 10:1 in 860 g Wasser innerhalb von 30 min redispergiert.
Diese Coating Dispersion wurde in einem Hüttlin Kugel Coater (HKC 5) auf ein Trägergranulat aufgetragen.
Als Trägergranulat wurden 4000 g Düngerpellets mit einem mittleren Durchmesser von 1,25 mm eingesetzt. Das Trägergranulat wurde in dem HKC 5 vorgewärmt bis 40°C Produkttemperatur, dann wurde ein Gemisch einer 20 Gew.-%igen Fenpropimorph Emulsion (36,7 g Wirkstoff) und einer 45 Gew.-%igen Epoxiconazol Suspension (5,7 g Wirkstoff) auf das Granulat aufgesprüht.

Auf das mit den Wirkstoffen Epoxiconazol und Fenpropimorph beschichtete Trägergranulat wurde die Polymerdispersion als Hülle aufgetragen.

Der Coatingprozeß wurde unter folgenden Bedingungen durchgeführt:

| | |
|---|---|
| 3 Düsen (Dreistoffdüsen) | 0,8 mm |
| Einwaage an Trägergranulat | 4000 g |
| Luftmenge | 400 m³/h |
| Zulufttemperatur | 50°C |
| Produkttemperatur | 40-42°C |
| Ablufttemperatur | 37,5-40°C |
| Sprühart | kontinuierlich |
| Sprühdruck | 1 bar |
| Sprührate (für Wirkstoffe und Hüllpolymer) | 12 g/min |
| Prozeßdauer | 90 min |
| Nachtrocknungszeit bis 50°C Produkttemperatur | 15 min |

Es bildete sich ein gleichmäßig glatter Überzug aus.

Die Freisetzungsrate der Wirkstoffe wurde in einer Elutionsapparatur bestimmt. Hierzu werden 30 g des Trägergranulates in ein Syphongefäß mit Fritte eingewogen. Durch das Gefäß wird in 24 h eine Menge von 3750 g Wasser gepumpt und aufgefangen. In der aufgefangenen Flüssigkeit wurden 124 mg Fenpropimorph und 18,5 mg Epoxiconazol nachgewiesen.

### Beispiel 7

In einem Rührgefäß wurden unter Rühren 130 g redispergierbares Pulver, bestehend aus Polyvinylacetat und Polyvinylpyrrolidon (Kollidon® 30, Fa. BASF) im Gewichtsverhältnis 12:1 in 295 g Wasser innerhalb von 45 min redispergiert.
Diese Coating Dispersion wurde in einem Hüttlin Kugel Coater (HKC 5) auf ein Trägergranulat aufgetragen.
Als Trägergranulat wurden 3240 g Düngerpellets mit einem mittleren Durchmesser von 1,3 mm eingesetzt. Das Trägergranulat wurde in dem HKC 5 vorgewärmt bis 40°C Produkttemperatur, dann wurde ein Gemisch einer 20 Gew.-%igen Fenpropimorph Emulsion (65,6 g Wirkstoff) und einer 45 Gew.-%igen Epoxiconazol Suspension (21,9 g Wirkstoff) auf das Granulat aufgesprüht.

Auf das mit den Wirkstoffen Epoxiconazol und Fenpropimorph beschichtete Trägergranulat wurde die Polymerdispersion als Hülle aufgetragen.

Der Coatingprozeß wurde unter folgenden Bedingungen durchgeführt:

| | |
|---|---|
| 3 Düsen (Dreistoffdüsen) | 1,4 mm |
| Einwaage an Trägergranulat | 3240 g |
| Luftmenge | 280-300 m³/h |
| Zulufttemperatur | 60°C |
| Produkttemperatur | 43-45°C |
| Ablufttemperatur | 37,5-40°C |
| Sprühart | kontinuierlich |
| Sprühdruck | 0,8 bar |
| Sprührate (für Wirkstoffe und Hüllpolymer) | 19 g/min |
| Prozeßdauer | 23 min |
| Nachtrocknungszeit bis 50°C Produkttemperatur | 15 min |

Es bildete sich ein gleichmäßig glatter Überzug aus.

Die Freisetzungsrate der Wirkstoffe wurde in einer Elutionsapparatur bestimmt. Hierzu werden 30 g des Trägergranulates in ein Syphongefäß mit Fritte eingewogen. Durch das Gefäß wird in 24 h eine Menge von 3980 g Wasser gepumpt und aufgefangen. In der aufgefangenen Flüssigkeit wurden 148 mg Fenpropimorph und 19 mg Epoxiconazol nachgewiesen.

Das gecoatete Produkt wurde im Oktober mit Getreidesaatgut (Winterweizen) ausgebracht. Im Mittel wurden pro Saatgutkorn ca. 3 fungizide Wirkstoffpellets unter der Erde abgelegt. In den wachsenden Getreidepflanzen wurde zwei Monate, bzw. sechs Monate nach Ausbringung Epoxiconazol nachgewiesen. Zwei Monate nach der Aussaat konnten 0,038 mg/kg Grünmasse, sechs Monate nach der Ausbringung noch 0,022 mg/kg Grünmasse nachgewiesen werden.

Damit konnte gezeigt werden, daß eine kontrollierte Abgabe des Wirkstoffes Epoxiconazol durch den Polyvinylacetat/Polyvinylpyrrolidon-Überzug über mindestens 6 Monate im Boden gewährleistet werden kann.

## Patentansprüche

1. Verwendung von redispergierbaren Polymerpulvern oder Polymergranulaten aus
a) 10 bis 95 Gew.-% Polyvinylacetat,
b) 5 bis 90 Gew.-% eines N-Vinylpyrrolidon-haltigen Polymers,
c) 0 bis 20 Gew.-% eines weiteren wasserlöslichen oder wasserquellbaren Stoffes und
d) 0 bis 20 Gew.-% eines wasserunlöslichen Puderungsmittels sowie gegebenenfalls
e) weiteren Zusatzstoffen,
zum Überziehen von pharmazeutischen oder agrochemischen Darreichungsformen.

2. Verwendung von redispergierbaren Polymerpulvern oder Polymergranulaten nach Anspruch 1, die als Komponente b) ein Polymer aus der Gruppe Polyvinylpyrrolidon, Vinylacetat-Vinylpyrrolidon Copolymere und Mischungen davon enthalten.

3. Verwendung von redispergierbaren Polymerpulvern oder Polymergranulaten nach den Ansprüchen 1 und 2, die als Komponente c) ein Polymer aus der Gruppe Cellulosederivate, Acrylat-Methacrylat Copolymere und Polyvinylalkohole oder Mischungen davon enthalten.

4. Verwendung von redispergierbaren Polymerpulvern oder Polymergranulaten nach den Ansprüchen 1 bis 3, die ein wasserunlösliches Puderungsmittel aus der Gruppe Cellulose, disperse Kieselsäure, Talkum, Bentonit, Magnesiumstearat und Caiciumphosphat oder Mischungen davon enthalten.

5. Verwendung von redispergierbaren Polymerpulvern oder Polymergranulaten nach den Ansprüchen 1 bis 4, **dadurch gekennzeichnet, daß** sie in Form einer wäßrigen Dispersion auf die wirkstoffhaltige Darreichungsform aufgebracht werden.

6. Feste pharmazeutische und agrochemische Darreichungsformen mit einem wirkstoffhaltigen Kern und einem Überzug aus
a) 10 bis 95 Gew.-% Polyvinylacetat,
b) 5 bis 90 Gew.-% eines N-Vinylpyrrolidon-haltigen Polymers,
c) 0 bis 20 Gew.-% eines weiteren wasserlöslichen oder wasserquellbaren Stoffes und
d) 0 bis 20 Gew.-% eines wasserunlöslichen Puderungsmittels sowie gegebenenfalls
e) weiteren Zusatzstoffen.

7. Feste pharmazeutische Darreichungsformen nach Anspruch 6, aus denen die Wirkstoffe innerhalb einer Zeit von 0,25 bis 24 Stunden freigesetzt werden.

8. Feste agrochemische Darreichungsformen nach Anspruch 6, aus denen die Wirkstoffe innerhalb einer Zeit von 6 Wochen bis 6 Monaten freigesetzt werden.

## Claims

1. The use of redispersible polymer powders or polymer granules consisting of
a) 10 - 95% by weight of polyvinyl acetate,
b) 5 - 90% by weight of an N-vinylpyrrolidone-containing polymer,
c) 0 - 20% by weight of another water-soluble or water-swellable substance and
d) 0 - 20% by weight of a water-insoluble dusting agent with or without
e) other additives,
for coating pharmaceutical or agrochemical use forms.

2. The use of redispersible polymer powders or polymer granules as claimed in claim 1 which comprise as component b) a polymer from the group of polyvinylpyrrolidone, vinyl acetate/vinylpyrrolidone copolymers and mixtures thereof.

3. The use of redispersible polymer powders or polymer granules as claimed in claims 1 and 2 which comprise as component c) a polymer from the group of cellulose derivatives, acrylate/methacrylate copolymers and polyvinyl alcohols or mixtures thereof.

4. The use of redispersible polymer powders or polymer granules as claimed in any of claims 1 to 3 which comprise a water-insoluble dusting agent from the group of cellulose, disperse silica, talc, bentonite, magnesium stearate and calcium phosphate or mixtures thereof.

5. The use of redispersible polymer powders or polymer granules as claimed in any of claims 1 to 4 which are applied in the form of an aqueous dispersion to the use form containing active ingredients.

6. A solid pharmaceutical or agrochemical use form having a core containing active ingredients and having a coating consisting of
a) 10 - 95% by weight of polyvinyl acetate,
b) 5 - 90% by weight of an N-vinylpyrrolidone-containing polymer,
c) 0 - 20% by weight of another water-soluble or water-swellable substance and
d) 0 - 20% by weight of a water-insoluble dusting agent with or without
e) other additives.

7. A solid pharmaceutical use form as claimed in claim 6, from which the active ingredients are released in a period of from 0.25 to 24 hours.

8. A solid agrochemical use form as claimed in claim 6, from which the active ingredients are released in a period of from 6 weeks to 6 months.

## Revendications

1. Utilisation de poudres polymères ou de granulés polymères redispersables comportant
a) 10 à 95 % en poids de poly(acétate de vinyle),
b) 5 à 90 % en poids d'un polymère contenant de la N-vinylpyrrolidone,
c) 0 à 20 % en poids d'une autre substance soluble dans l'eau ou expansible dans l'eau, et
d) 0 à 20 % en poids d'un agent de poudrage insoluble dans l'eau,
e) d'autres additifs,
pour l'enrobage de formes d'administration pharmaceutiques ou agrochimiques.

2. Utilisation de poudres polymères ou de granulés polymères redispersables selon la revendication 1, qui contiennent comme composant b) un polymère du groupe de la poly(pyrrolidone de vinyle), des copolymères d'acétate de vinyle-pyrrolidone de vinyle, et leurs mélanges.

3. Utilisation de poudres polymères ou de granulés polymères redispersables selon les revendications 1 ou 2, qui contiennent comme composant c) un polymère du groupe des dérivés de cellulose, des copolymères d'acrylate-méthacrylate et des poly(alcools de vinyle), ou leurs mélanges.

4. Utilisation de poudres polymères ou de granulés polymères redispersables selon les revendications 1 à 3, qui contiennent un agent de poudrage insoluble dans l'eau choisi dans le groupe de la cellulose, de l'acide silicique dispersé, du talc, de la bentonite, du stéarate de magnésium et du phosphate de calcium, ou de leurs mélanges.

5. Utilisation de poudres polymères ou de granulés polymères redispersables selon les revendications 1 à 4, **caractérisée par le fait qu'**ils sont appliqués sous la forme d'une dispersion aqueuse sur la forme d'administration contenant la substance active.

6. Formes d'administration pharmaceutiques ou agrochimiques solides, ayant un noyau contenant des substances actives et un revêtement de
a) 10 à 95 % en poids de poly(acétate de vinyle),
b) 5 à 90 % en poids d'un polymère contenant de la N-vinylpyrrolidone,
c) 0 à 20 % en poids d'une autre substance soluble dans l'eau ou expansible dans l'eau, et
d) 0 à 20 % en poids d'un agent de poudrage insoluble dans l'eau,
ainsi qu'éventuellement
e) d'autres additifs.

7. Formes d'administration pharmaceutiques solides selon la revendication 6, à partir desquelles les substances actives sont libérées en un temps de 0,25 à 24 heures.

8. Formes d'administration agrochimiques solides selon la revendication 6, à partir desquelles les substances actives sont libérées en un temps de 6 semaines à 6 mois.
